(19)

Europäisches
Patentamt

European
Patent Office

Office européen
des brevets

(11) **EP 1 978 155 A1**

(12) **EUROPÄISCHE PATENTANMELDUNG**

(43) Veröffentlichungstag:
 **08.10.2008 Patentblatt 2008/41**

(51) Int Cl.:
 *D21F 11/14* *(2006.01)*

(21) Anmeldenummer: **08006830.7**

(22) Anmeldetag: **04.04.2008**

(84) Benannte Vertragsstaaten:
 **AT BE BG CH CY CZ DE DK EE ES FI FR GB GR HR HU IE IS IT LI LT LU LV MC MT NL NO PL PT RO SE SI SK TR**
 Benannte Erstreckungsstaaten:
 **AL BA MK RS**

(30) Priorität: **05.04.2007 DE 102007016959**

(71) Anmelder: **McAirlaid's Vliesstoffe GmbH & Co. KG 48565 Steinfurt (DE)**

(72) Erfinder: **Schmidt, Andreas 37115 Duderstadt (DE)**

(74) Vertreter: **Christophersen & Partner Patentanwälte Feldstrasse 73 40479 Düsseldorf (DE)**

(54) **Faserstoffbahn**

(57)     Es wird eine Faserstoffbahn aus Zellstoff-Fasern beansprucht, bei der die Fasern unter Erzeugung eines Prägemusters Im Druckbereich unter Fusion der Faserkörper bindemittelfrei punkt- oder linienförmig kalandriert und verbunden sind, dadurch gekennzeichnet, dass chemisch behandelte Zellstoff-Fasern verwendet werden. Die Fäserstoffbahn eignet sich zum Einsatz In Hygieneartikeln und als Filtermaterial.

FIG. 1

EP 1 978 155 A1

**Beschreibung**

Die Erfindung betrifft eine Faserstoffbahn aus Zellstoff-Fasern, bei welcher die Fasern unter Erzeugung eines Prägemusters Druckbereichen unter Fusion der Faserkörper punkt- oder linienförmig kalandriert und verbunden sind. Ferner betrifft die Erfindung die Verwendung der Faserstoffbahn zur Herstellung von Hygieneartikeln sowie Hygieneartikel, welche die Faserstoffbahn als Saugkern enthalten und die Verwendung der Faserstoffbahn als Filtermaterialien.

Im Hygienebereich finden Faserstoffbahnen aus Zellstoff-Fasern eine breite Anwendung. Die Faserstoffbahnen dienen als Saugkerne zur Aufnahme von Körperflüssigkeiten. Sie haben die Aufgabe, die Körperflüssigkeiten schnell aufzunehmen und dauerhaft zu absorbieren. Eine weitere Eigenschaft einer Zellstoffbahn ist die Durchleitung von Flüssigkeit oder Luft in darunter liegende Strukturen. Da die Faserstoffbahnen vielfach direkten Körperkontakt haben, sollten sie möglichst gut körperverträglich sein und die eintretende Flüssigkeit gut verteilt aufnehmen. Zellstoff hat weiterhin den Vorteil, dass er biologisch abbaubar ist, d.h. er kann auf entsprechenden Deponien entsorgt werden, wo er rückstandslos verrottet. Für ihren Einsatz in Hygieneartikeln sollen die Faserstoffbahnen hoch saugfähig oder gut durchleitend, weich und als Bahn verarbeitbar sein. Zur Erhöhung der Absorptionskapazität werden der Faserstoffmatrix häufig so genannte Superabsorber, d.h. Polymere, die unter Bildung von Hydrogelen Wasser aufnehmen können, zugesetzt. Der Einsatz der Faserstoffbahnen im Hygienebereich macht es erforderlich, dass die zur Herstellung der Hygieneartikel verwendeten Materialien Additive und sonstige Hilfsstoffe nur in sehr geringem Umfang enthalten. Für die Verarbeitung der Faserstoffbahnen zu Saugkemen werden diese üblicherweise in Form von Zellstoff-Vliesen und Airlaids eingesetzt. Der Verbund der einzelnen Zellstoff-Fasern wird über Bindemittel erreicht.

Die Herstellung von bindemittelfreien Faserstoffbahnen ist ebenfalls bekannt. So wird im europäischen Patent 1 032 342 B1 ein Verfahren zur Herstellung einer aus Zellstoff-Fasern oder Zellstoff-Pulpe oder Zellstoff-Karton bestehenden, ohne Verwendung von zusätzlichen Bindemitteln hergestellten, saugfähigen und rollbaren Faserstoffbahnen mit einer Reißfestigkeit von wenigstens 0,12 kN/m offenbart. In dem beschriebenen Verfahren wird eine regellose Zellstoff-Faserlage vorgelegt und unter einem relativ niedrigen Druck vorverdichtet, wobei ein lockeres Vlies mit geringer Dichte und Reißfestigkeit erzeugt wird. Dieses Vlies, das einen Feuchtigkeitsgehalte bis zu 5 Gewichtsprozent aufweist, wird In den Spalt eines Kalanderrollen-Paares eingeführt, wobei ein Muster von punkt- oder linienförmigen Druckbereichen unter einem relativ höheren Druck erzeugt wird, so dass die regellos liegenden Fasern aufeinander gedrückt werden. Die Fasern werden in dem Kalanderrollen-Paar unter einem Druck von 250 - 600 MPa aufeinander gedrückt, so dass eine nicht-lösende Fusion der Fasern erfolgt und auf der Faserstoffbahn ein Prägemuster erzeugt wird. Zur Herstellung der aus dem Stand der Technik bekannten Faserstoffbahnen werden handelsübliche Zellulosefasern eingesetzt, die beispielsweise unter der Fachbezeichung "Fluff Pulp" bekannt sind. In dieser Einsatzform liegen die Zellulosefasern in gestreckter Form vor.

Der Einsatz im Hygienebereich macht es erforderlich, dass nur geringen Mengen an Additiven zur Erhöhung der Absorptionskapazität enthalten sind. Aus dem US-Patent 4,898,642 sind Fasern bekannt, die verdrillt und chemisch versteift sind. Diese Fasern weisen ein höheres Volumen zur Aufnahme der Flüssigkeit verglichen mit gestreckten Fasern auf. Die Verarbeitung dieser Fasern zu Hygieneartikeln erfordert es jedoch, dass ein Bindemittel eingesetzt wird. Ein Hygieneartikel, welches diese Fasern enthält, ist beispielsweise im europäischen Patent 0 598 823 offenbart. Durch den Einsatz des Bindemittels geht ein Teil der durch die verdrehte Struktur erreichte höhere Aufnahmegeschwindigkeit wieder verloren. Ferner ist das Bindemittel ein Additiv, das die Hautverträglichkeit des Materials in einem Hygieneartikel beeinträchtigen kann.

Der vorliegenden Erfindung lag die Aufgabe zugrunde, eine Faserstoffbahn herzustellen, die sich für den Einsatz in Hygieneartikeln eignet und die eine gegenüber dem Stand der Technik bekannten Zellulosefasern erhöhte Absorptionsgeschwindigkeit. Bei einem möglichen Einsatz als Filtermaterial sollte die Faserstoffbahn eine gute Durchleitungsgeschwindigkeit bzw. geringen Druckabfall aufweisen. Ferner sollte zum Erreichen dieser Eigenschaften auf den Zusatz von chemischen Additiven möglichst ganz verzichtet werden, da es zu Unverträglichkeiten der Haut kommen kann und die Gefahr besteht, dass die Additive bei Einsatz als Filter auf das zu filternde Medium übertreten.

Gegenstand der vorliegenden Erfindung ist eine Faserstoffbahn aus Zellstoff-Fasern, bei der die Fasern bindemittelfrei unter Erzeugung eines Prägemusters im Druckbereich unter Fusion der Faserkörper punkt- oder linienförmig kalandriert und verbunden sind, dadurch gekennzeichnet, dass chemisch behandelte Zellstoff-Fasern verwendet werden.

Überraschenderweise wurde festgestellt, dass durch die veränderte Struktur der Zellstoff-Fasern die Aufnahmegeschwindigkeit und Durchleitungsgeschwindigkeit der aufzunehmen Körperflüssigkeiten deutlich erhöht werden kann. Die Körperflüssigkeiten werden schnell aufgenommen oder durchgeleitet, d.h. abtransportiert, und absorbiert (eigentlich absorbieren gecurlte oder mercerisierte Fasern weniger als normaler Zellstoff). Ebenfalls wurde festgestellt, dass in Verbindung mit superabsorbierenden Polymeren nach der Durchleitung der Flüssigkeit in tiefere Strukturen die Faserbahn deutlich trockener ist als bei einer Fesaerbahn mit unbehandelten Fasern. Durch die Fusion der Faserkörper unter Druck wird punkt- oder linienförmig eine Verbindung zwischen den Fasern hergestellt, so dass die Fa-

serstoffbahn einen festen Verbund erhält, ohne dass sich das Volumen der Zellstoff-Fasern wesentlich verringert. Es ist nicht erforderlich, die Zellstoff-Fasern noch mit Bindemitteln oder anderen Additiven zu beaufschlagen, um die Reißfestigkeit, die in Hygieneartikeln notwendig ist, zu erreichen.

Im Rahmen der vorliegenden Erfindung werden chemisch behandelte Zellstoff-Fasem eingesetzt. Die chemische Behandlung kann beispielsweise Curlen oder Mercerisieren sein. Gecurlte Fasern sind um ihre Achse verdreht und/oder entlang einer Achse aufgewickelt sie haben ein gekräuseltes Aussehen. Besonders bevorzugte Fasern weisen einen Curl-Index von mindestens 0,3, insbesondere von mindestens 0,5 auf.

Der Curl-Index bezeichnet eine zweidimensionale Messung der Kräuselung und wird durch Betrachten der Faser in einer zweidimensionlaen Ebene bestimmt, wobei die projizierte Länge der Faser als die längste Dimension eines Rechtecks, das die Faser umschreibt. $L_R$, und die tatsächliche Länge der Faser, $L_A$, gemessen werden. Der Curl-Index wird gemäß der nachfolgenden Gleichung berechnet:

$$\text{Curl-Index} = (L_A/L_R - 1.$$

Die Werte $L_A$ und $L_R$ werden durch eine Bildanalyse bestimmt. Die Bestimmungsmethode ist dem Fachmann bekannt werden u. a. in "Application of Image Analysis to Pulp Fibre Characterization: Part 1", von B. D. Jordan and D. H. Page, S. 104.114, Canadian Pulp and Paper Association (Montreal, Quebec, Kanada) sowie im experimentellen Teil der EP0429112 beschrieben.

In einer möglichen Ausführungsform sind die gecurlten Fasern chemisch versteift, d.h. die Fasern wurden chemisch behandelt, um ihre Festigkeit bzw. Steifigkeit unter trockenen und auch besonders unter feuchten Bedingungen zu erhöhen. Die Verfestigung der Zellstoff-Fasern kann intramolekular und/oder intermolekular erfolgen, d.h. innerhalb einer einzelnen Zellstoff-Faser bzw. eines einzelnen Zellulose-Moleküls oder zwischen unterschiedlichen Molekülen. Auf diese Weise ergibt sich durch die Behandlung mit dem Versteifungsmittel eine verdrehte und/oder gewickelte Konfiguration. Geeignete Versteifungsmittel für ZelluloseFasern sind solche, die eine chemische Vernetzung durch Reaktion der Hydroxylgruppen in den Zellulose-Molekülen bewirken, wie monomere Vernetzungsmittel. Beispiele für monomere Vernetzungsmittel sind Dialdehyde mit 2 - 8 Kohlenstoffatomen, Monoaldehyde mit 2 bis 8 Kohlenstoffatomen und einer weiteren Säuregruppe oder Dicarbonsäuren mit 2 bis 10 Kohlenstoffatomen. Diese Verbindungen können mit mindestens zwei Hydroxylgruppen im Molekül der Zellulosekette oder einer benachbarten Kette in einer Faser reagieren. Besonders geeignete Vernetzungsmittel sind Acetaldehyd, Glyoxal, Glutaraldehyd, Glyoxalsäure, Oxalsäure, Malonsäure, Bernsteinsäure, Glutarsäure, Adipinsäure, Pimelinsäure, Korksäure, Azelainsäure, Sebacinsäure, Maleinsäure und Fumarsäure, Äpfelsäure, Weinsäure, $\alpha$-Ketoglutarsäure, Glutarsäure, Glutaminsäure, Asparaginsäure, Oxalessigsäure sowie auch Phthalsäure und Isophthalsäure. Polycarboxylate wie Zitronensäure, Tartrate, Succinate etc. , wie sie in der EP0429112 beschrieben werden, sind ebenfalls geeignet.

Eine weitere Möglichkeit, Zellulosefasern chemisch zu behandeln, ist das Merzerisieren. Hierbei werden die Zellulosefasern mit Lauge und ggf. weiteren Hilfsmitteln behandelt, wodurch inter- und intramolekulare Bindungen im Zellulosemolekül aufgebrochen werden und somit eine Entspiralisierung. Je nach Verfahrensweise, ob die Einwirkung der Lauge unter Zugspannung erfolgt oder nicht, werden auch Stretch-Fasern erhalten.

In einigen Anwendungsbereichen kann es erforderlich sein, dass die erfindungsgemäße Faserstoffbahn eine erhöhte Reißfestigkeit aufweisen muss. Für diese Anwendungen kann die Faserstoffbahn eine Armierungsbahn enthalten. Diese kann beispielsweise bei der Herstellung der Faserstoffbahn zwischen zwei Teil-Faserstoffbahnen aus Zellstoff-Fasern eingelegt und mitverarbeitet werden. Diese Armierungsbahn kann beispielsweise ein Gewebe, ein Gewirke oder eine Nonwoven-Bahn sein. Möglich sind auch durchgehende Folien mit in den Druckbereichen aufgerissenen oder löchrig geschmolzen Perforationen. Vorzugweise ist die Armierungsbahn biegsam.

Je nach Anwendungszweck kann die erfindungsgemäße Faserstoffbahn auch weitere Inhaltsstoffe enthalten, die dazu in der Lage sind, Flüssigkeiten, insbesondere Wundsekrete und Urin, aufzusaugen oder durchzuleiten. Neben den Zellulosefasern sind auch so genannte Superabsorber, Geruchsbinder, Geruchsstoffe, Aktivkohle oder andere Stoffe bindende Komponenten oder Stoffe abgebende Materialien, z. B. anorganische Absorptionsmittel, wie Zeolithe, sowie bakteriell wirkende Stoffe und/oder Pigmente, als Inhaltsstoffe möglich.

Die erfindungsgemäße Faserstoffbahn eignet sich zur Herstellung von Hygieneartikeln, wie Krankenauflagen, Windeln, Slipeinlagen, Inkontinenzprodukte, Einlagen für Lebensmittelverpackungen, Filtermaterial.

Die erfindungsmäße Faserstoffbahn eignet sich als Durchleitungslayer zur Verwendung in Hygieneartikeln wie Krankenauflagen, Windeln, Slipeinlagen, Inkontinenzprodukte; Einlagen für Lebensmittelverpakkungen, Filtermaterial.

Die erfindungsgemäße Faserstoffbahn eignet sich in Verbindung mit superabsorbierenden Polymeren als Durchleitungslayer mit deutlich reduziertem Feuchtigkeltsanteil in den Fasern oder der Faserbahn nach Flüssigkeitdurchleitung und Nachtrocknung durch den Superabsorber in der Faserbahn zur Verwendung in Hygieneartikeln wie Krankenauflagen, Windeln, Slipeinlagen, Inkontinenzprodukte, Einlagen für Lebensmittelverpackungen, Filtermaterial.

Ein weiterer Gegenstand betrifft demgemäß die Verwendung der Faserstoffbahn wie sie oben beschrieben wurde in Hygieneartikeln, wie Windeln, Krankenauflagen, Slipeinlagen, inkontinenzprodukte, Lebensmittelverpackungen, als Filtermaterial etc.

Ein weiterer Gegenstand der vorliegenden Erfindung ist eine mehrlagig aufgebaute Saugmatte mit einer für Flüssigkeiten undurchlässigen Basisschicht und einer Schicht aus einem absorbierenden Material, die dadurch gekennzeichnet ist, dass die Schicht aus einem absorbierenden Material eine Faserstoffbahn aus chemisch veränderten Zellstoff-Fasern ist und bei welcher die Fasern unter Erzeugung eines Prägemusters in Druckbereichen unter Fusion der Faserkörper punkt- oder linienförmig kalandriert und verbunden sind.

Bei der Verwendung in einer mehrlagig aufgebauten Saugmatte kann die erfindungsgemäße Faserstoff unterschiedliche Funktionen haben. In einer möglichen Ausgestaltung weist die Saugmatte eine Schicht aus einem absorbierenden Material auf und eine für Flüssigkeiten undurchlässige Basisschicht. In dieser Ausführungsform hat die Schicht aus dem absorbierenden Material die Aufgabe, die aufzunehmende Flüssigkeit aufzunehmen, zu verteilen, so dass die Flüssigkeit sich nicht nur in bestimmten Bereichen der Saugmatte befindet sondern gleichmäßig verteilt ist, und die Flüssigkeit auch zurückzuhalten, d.h. zu speichern. Das Rückhalten bzw. die Speicherung der Flüssigkeit soll verhindern, dass die aufgenommene Flüssigkeit wieder aus der Saugmatte hinaustritt. Zur Verbesserung der Speicherkapazität hat es sich als vorteilhaft erwiesen, wenn die Schicht aus absorbierenden Material Zusatzstoffe, wie Superabsorber oder anorganische Absorptionsmittel enthält.

In einer weiteren möglichen Ausführungsform weist die Saugmatte mehr als zwei Schichten auf, insbesondere eine die Flüssigkeit aufnehmende und verteilende Schicht (aquistion and distribution layer) und eine die Flüssigkeit speichernde Schicht. Die erfindungsgemäße Faserstoffbahn stellt in dieser Ausführungsform die die Flüssigkeit aufnehmende und verteilende Schicht dar. Sie hat die Aufgabe, die Flüssigkeit möglichst schnell von dem diese Flüssigkeit abgebenden Gegenstand bzw. Körper abzutransportieren und großflächig über die Saugmatte zu verteilen. Die Speicherung der Flüssigkeit erfolgt in einer darunter liegenden Schicht, die üblicherweise Zellulose und ähnliche Materialien in verfestigter Form oder als Fluff Pulp sowie Absorptionsmittel und ggf. weitere der oben genannten Zusatzstoffe enthält. Die Speicherung der Flüssigkeit kann auch teilweise oder ganz mit absorbierendem Material, wie Superabsorber innerhalb der Faserbahn geschehen, um eine Rest- und/ oder Nachtrocknung der Faserbahn zu erreichen. In Verbindung mit Superabsorbern ändert sich zusätzlich nach der ersten Flüssigkeitsdurchleitung und Nachtrocknung der Faserbahn das Volumen der Fasenbahn. Die Flüssigkeitsdurchleitung bei späteren Flüssigkeitszugaben wird dadurch erhöht. Der Nachtrocknungseffekt kann bei weiteren Flüssigkeitszugaben geringer ausfallen als bei der ersten Zugabe.

Um den direkten Kontakt der Körperoberfläche mit dem mit dem hier beschriebenen Material zu vermeiden weist die Saugmatte in einer optionalen Ausführungsform über der absorbierenden und Flüssigkeit verteilenden Schicht eine für Flüssigkeiten durchlässige Deckschicht auf. Besonders vorteilhaft ist es, wenn diese Deckschicht aus einem hydrophilen Material besteht. Das hydrophile Material erleichtert das Hindurchtreten von wässrigen Flüssigkeiten und somit die Aufnahme dieser Flüssigkeit durch das absorbierende Material. Die Deckschicht und/oder das absorbierende Material können zusätzlich noch antibakteriell wirkende Stoffe, die eine Keimvermehrung verhindern bzw. minimieren, enthalten, z.B. durch eine Bedampfung mit Metallen wie Silber oder Aluminium.

Insbesondere wenn die erfindungsgemäße Saugmatte im Hygienebereich eingesetzt werden soll, ist es bevorzugt, wenn diese zu ihren Seiten hin einen Auslaufschutz aufweist. Dazu wird in einer möglichen Ausführungsform die Basisschicht etwas größer dimensioniert als die absorbierende Schicht, so dass die Basisschicht um die Ränder der absorbierenden Schicht gefaltet und diese Ränder umschließen kann, so dass keine aufgenommene Flüssigkeit seitlich, d.h. über die Seitenränder, austreten kann.

Ein weiterer Einsatzbereich der erfindungsgemäßen Faserstoffbahn ist die Verwendung als Filtermittel zum Abscheiden von festen Teilchen aus einer Flüssigkeit oder einem Gas.

Ein weiterer Gegenstand der vorliegenden Erfindung ist demgemäß ein Filtermaterial enthaltend eine Faserstoffbahn aus Zellstoff-Fasern, bei der die Fasern unter Erzeugung eines Prägemusters im Druckbereich unter Fusion der Faserkörper bindemittelfrei punkt- oder linienförmig kalandriert und verbunden sind, dadurch gekennzeichnet, dass chemisch behandelte Zellstoff-Fasern verwendet werden.

Die erfindungsgemäße Faserstoffbahn wird in den beigefügten Figuren näher erläutert, es zeigen

Fig. 1    in perspektivischer Darstellung einen Abschnitt einer möglichen Ausführungsform der Faserstoffbahn,

Fig. 2    in perspektivischer Darstellung einen Abschnitt einer weiteren Ausführungsform der Faserstoffbahn, und

Fig. 3    in perspektivischer Darstellung einen Abschnitt eine mögliche Ausgestaltung der Ausführungsform aus Fig. 1.

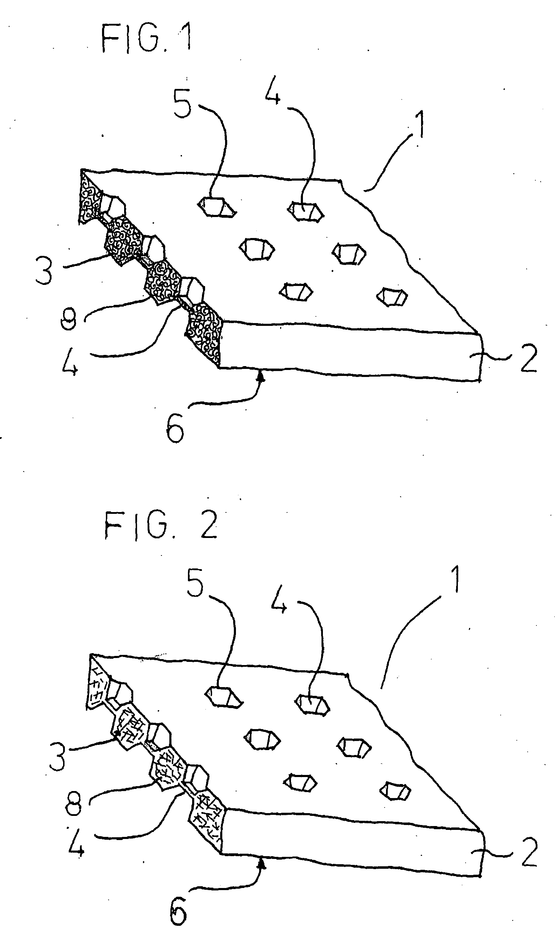

In Figur 1 ist ein Abschnitt 2 der Faserstoffbahn 1 dargestellt. Diese besteht aus gecurlten Zellstofffasern 3, die in Prägebereichen 4 miteinander fusioniert sind.

Die Prägebereiche 4 sind vorzugsweise so angeordnet, dass die Prägebereiche der Ober- und Unterseite 5 und 6 der Bahn sich gegenüberliegen, wodurch im Prägebereich ein sehr schmaler Steg 7 von miteinander verbundenen Zellstofffasern vorliegt. Die Faserstoffbahn weist demnach dünne Prägebereiche 4 auf, die sich jeweils mit etwas aufgebauschten lockeren Bereichen 8 abwechseln. Eine innige Verbindung zwischen den Zellstofffasern 3 besteht in den lockeren Bereichen 8 nicht. In diesen Bereichen kommen die vorteilhaften Eigenschaften Zellstofffasern zum Tragen.

In Figur 2 ist eine weitere Ausführungsform der vorliegenden Erfindung dargestellt. Im Unterschied zur Faserstoffbahn aus Figur 1 enthält die Faserstoffbahn aus Figur 2 glatte, d.h. mercerisierte Fasern.

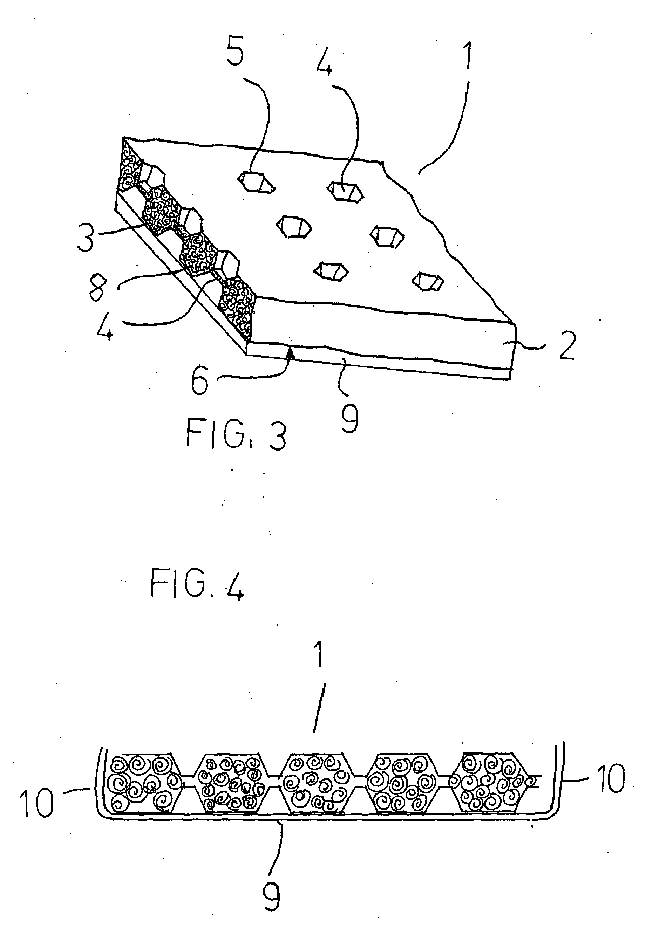

In Figur 3 ist eine mögliche Ausgestaltung der Faserstoffbahn aus Figur 1 dargestellt, bei der ein Abschnitt 2 der Faserstoffbahn 1 mit einem Folienabschnitt 9 belegt ist. Der Folienabschnitt 9 stellt die Basisschicht dar und die Faserstoffbahn die absorbierende Schicht. Die Verbindung zwischen dem Abschnitt 2 und dem Folienabschnitt 9 kann mit einem Klebstoff erzielt werden. Nach Gebrauch kann die Folie 9 samt dem Klebstoff von dem Faserstoffbahnabschnitt abgezogen werden, um ein sortenreines Recycling zu ermöglichen. Es kann auch eine mechanische Verbindung dadurch hergestellt werden, dass die Folle 9 an weiteren, hier nicht dargestellten Prägestellen perforiert ist, wobei Ausstanzuhgen der Folle 9 in den lockeren Zellstoff 3 hineingedrückt und dort verankert werden.

Figur 4 ist eine weitere Ausgestaltung der Faserstoffbahn aus Figur 3, bei der die Folie 9, d.h. die Basisschicht die absorbierende Schicht an zwei Rändern 10 umschließt.

Bezugszeichenliste

1 Faserstoffbahn
2 Abschnitt der Faserstoffbahn
3 Zellstofffaisern
4 Prägebereiche
5 Oberseite der Faserstoffbahn 1
6 Unterseite der Faserstoffbahn 1
7 Steg
8 lockerer Bereich
9 Folie
10 Rand

**Patentansprüche**

1. Faserstöffbahn aus Zellstoff-Fasern, bei der die Fasern unter Erzeugung eines Prägemusters im Druckbereich unter Fusion der Faserkörper bindemittelfrei punkt- oder linienförmig kalandriert und verbunden sind, **dadurch gekennzeichnet, dass** chemisch behandelte Zellstoff-Fasern verwendet werden.

2. Faserstoffbahn nach Anspruch 1, **dadurch gekennzeichnet, dass** die Zellulosefasern gecurlt oder mercerisiert sind.

3. Faserstoffbahn nach Anspruch 2 **dadurch gekennzeichnet, dass** die Fasern gecurlt sind und der Curl-Index mindestens 0,3 beträgt.

4. Faserstoffbahn nach einem der Ansprüche 1 bis 3, **dadurch gekennzeichnet, dass** die Zellstoff-Fasern chemisch versteift sind.

5. Faserstoffbahn nach Anspruch 4, **dadurch gekennzeichnet, dass** die chemische Versteifung durch Vernetzung der Zellulosefasern erfolgt.

6. Faserstoffbahn nach einem der Ansprüche 1 bis 5, **dadurch gekennzeichnet, dass** Superabsorber, Geruchsbinder, Geruchsstoffe, Aktivkohle, anorganische Absorptionsmittel, wie Zeolithe, bakteriell wirkende Stoffe und/oder Pigmente enthalten sind.

7. Faserstoffbahn nach einem der Ansprüche 1 bis 6, **dadurch gekennzeichnet, dass** sie eine Armierungsbahn aufweist.

8. Saugeinlage mit einer für Flüssigkeiten undurchlässigen Basisschicht und einer Schicht aus einem absorbierenden Material aus Zellstoff-Fasern, bei welcher die Fasern unter Erzeugung eines Prägemusters im Druckbereich unter Fusion der Faserkörper punkt- oder linienförmig kalandriert und verbunden sind, **dadurch gekennzeichnet, dass** die Zellstoff-Fasern um die eigene Achse verdrillt und/oder in Richtung ihrer Längsachse gewickelt sind

9. Saugeinlage nach Anspruch 8, **dadurch gekennzeichnet, dass** die Basisschicht die absorbierende Schicht an mindestens zwei Rändern umschließt.

10. Saugeinlage nach Anspruch 8 oder 9, **dadurch gekennzeichnet, dass** über der absorbierenden Schicht eine für Flüssigkeiten durchlässige Deckschicht angeordnet ist.

11. Verwendung der Saugeinlage nach einem der Ansprüche 8 bis 10 als Hygieneartikel, im Lebensmittelbereich oder als Filtermittel.

12. Verwendung nach Anspruch 11, **dadurch gekennzeichnet, dass** die Saugeinlage als Windel, Krankenunterlage, Wundverband, in Lebensmittelverpackungen, eingesetzt wird.

13. Filtermaterial enthaltend eine Faserstoffbahn aus Zellstoff-Fasern, bei der die Fasern unter Erzeugung eines Prägemusters im Druckbereich unter Fusion der Faserkörper bindemittelfrei punkt- oder linienförmig kalandriert und verbunden sind, **dadurch gekennzeichnet, dass** chemisch behandelte Zellstoff-Fasern verwendet werden.

FIG. 1

FIG. 2

FIG. 3

FIG. 4

**Europäisches**
**Patentamt**

**EUROPÄISCHER RECHERCHENBERICHT**

Nummer der Anmeldung

EP 08 00 6830

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 101 44 128 A1 (HARTMANN PAUL AG [DE]) 27. März 2003 (2003-03-27) * Zusammenfassung * * Absatz [0002] * * Absätze [0007], [0008] * * Absatz [0014] * * Absätze [0018] - [0026] * * Abbildungen * ----- | 1-13 | INV. D21F11/14 |
| X | DE 197 50 890 A1 (MAKSIMOW ALEXANDER [DE]) 27. Mai 1999 (1999-05-27) * Spalte 2, Zeile 66 - Spalte 3, Zeile 20 * * Spalte 4, Zeile 21 - Spalte 5, Zeile 23 * * Abbildungen * ----- | 1,6-13 | |
| A,D | US 4 898 642 A (MOORE DANNY R [US] ET AL) 6. Februar 1990 (1990-02-06) * Zusammenfassung * * Spalte 1, Zeilen 12-40 * * Spalte 8, Zeilen 31-55 * ----- | 1,8,13 | RECHERCHIERTE SACHGEBIETE (IPC) D21F A61F |
| A | US 5 531 728 A (LASH GLEN R [US]) 2. Juli 1996 (1996-07-02) * Zusammenfassung * * Spalte 9, Zeile 36 - Spalte 11, Zeile 15 * * Beispiel 1 * ----- | 1,8,13 | |

Der vorliegende Recherchenbericht wurde für alle Patentansprüche erstellt

| Recherchenort | Abschlußdatum der Recherche | Prüfer |
|---|---|---|
| München | 25. Juli 2008 | Pregetter, Mario |

**ANHANG ZUM EUROPÄISCHEN RECHERCHENBERICHT
ÜBER DIE EUROPÄISCHE PATENTANMELDUNG NR.**

EP 08 00 6830

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-07-2008

| Im Recherchenbericht angeführtes Patentdokument | Datum der Veröffentlichung | Mitglied(er) der Patentfamilie | Datum der Veröffentlichung |
|---|---|---|---|
| DE 10144128 A1 | 27-03-2003 | AT 287252 T<br>WO 03022194 A1<br>EP 1424982 A1<br>JP 2005501660 T<br>US 2004254552 A1 | 15-02-2005<br>20-03-2003<br>09-06-2004<br>20-01-2005<br>16-12-2004 |
| DE 19750890 A1 | 27-05-1999 | KEINE | |
| US 4898642 A | 06-02-1990 | KEINE | |
| US 5531728 A | 02-07-1996 | AT 158162 T<br>AU 658361 B2<br>AU 7183791 A<br>BR 9105936 A<br>CA 2069443 A1<br>CN 1053544 A<br>CS 9100136 A2<br>CZ 290313 B6<br>CZ 289768 B6<br>DE 69127689 D1<br>DE 69127689 T2<br>DK 512010 T3<br>EG 19130 A<br>EP 0512010 A1<br>ES 2106775 T3<br>FI 923341 A<br>GR 3024773 T3<br>HK 1003027 A1<br>HU 63760 A2<br>HU 215911 B<br>IE 910224 A1<br>JP 3696880 B2<br>JP 5503446 T<br>MA 22048 A1<br>NZ 236854 A<br>PH 30767 A<br>PL 288812 A1<br>PL 168533 B1<br>PT 96544 A<br>SG 72670 A1<br>RU 2090170 C1<br>TR 27249 A<br>WO 9111163 A1 | 15-10-1997<br>13-04-1995<br>21-08-1991<br>20-10-1992<br>24-07-1991<br>07-08-1991<br>13-08-1991<br>17-07-2002<br>17-04-2002<br>23-10-1997<br>15-01-1998<br>13-10-1997<br>30-07-1994<br>11-11-1992<br>16-11-1997<br>22-07-1992<br>31-12-1997<br>30-09-1998<br>28-10-1993<br>29-03-1999<br>31-07-1991<br>21-09-2005<br>10-06-1993<br>01-10-1991<br>27-09-1994<br>17-10-1997<br>27-01-1992<br>29-02-1996<br>15-10-1991<br>23-05-2000<br>20-09-1997<br>21-12-1994<br>08-08-1991 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1032342 B1
- US 4898642 A
- EP 0598823 A
- EP 0429112 A